# FASCICULE DE BREVET EUROPEEN

(11) **EP 2 222 658 B1**
(45) Date de publication et mention de la délivrance du brevet: **15.06.2011**
(21) Numéro de dépôt: 08862516.5
(22) Date de dépôt: 19.12.2008
(51) Int. Cl.: C07D 319/12

(54) **PROCÉDÉ D'OBTENTION DE LACTIDE**
VERFAHREN ZUR GEWINNUNG VON LACTID
METHOD FOR OBTAINING LACTIDE

(30) Priorité: 19.12.2007 EP 07024679
(43) Date de publication de la demande: 01.09.2010
(73) Titulaire: Futerro S.A., 7760 Escanaffles (BE)
(72) Inventeur: COSZACH, Philippe, B-7760 Escanaffles (BE); MARIAGE, Pierre-Antoine, B-7760 Escanaffles (BE)
(74) Mandataire: Leyder, Francis
(86) Numéro de dépôt international: PCT/EP2008/067994
(87) Numéro de publication internationale: WO 2009/077615

(56) Documents cités:
- WO-A-2005/056509
- GB-A- 2 331 986
- US-B1- 6 268 465

## Description

La présente invention concerne un procédé d'obtention de lactide par thermocraquage d'oligomères d'acide lactique.

Le lactide ou 3,6-dimethyl-1,4-dioxane-2,5-dione, est un dimère cyclique d'acide lactique. Le lactide est un intermédiaire dans de nombreux procédés industriels de production d'acide polylactique, un polyester biodégradable produit à partir de sources renouvelables. L'acide polylactique de haut poids moléculaire connaît de nombreuses applications dans les domaines tels que l'emballage alimentaire et le textile.

Une des voies de synthèse industrielle de lactide consiste en un procédé en deux étapes de polymérisation/dépolymérisation. L'acide lactique est d'abord polymérisé en des chaînes à faibles poids moléculaires (oligomères), ensuite ces chaînes sont chauffées pour les dépolymériser en lactide. Ce dernier est récupéré en phase vapeur.

Le document GB 2 331 986 A divulgue un procédé de préparation d'une lactone cyclique par chauffage de l'oligomère de l'acide hydroxy carboxylique correspondant sous pression réduite en présence d'un catalyseur et d'un composé phosphoré tel qu'un phosphite organique, un diphosphite ou un phosphonite comme stabilisant. Ce document ne divulgue pas l'utilisation de sel métallique de l'anion phosphite (PO₃)³ - comme catalyseur pour l'obtention de lactide à faible taux de racémisation.

Lors de la production d'oligomères d'acide lactique, du lactide est formé. Le document WO92/05167 divulgue que la concentration en lactide dans l'oligomère est fonction de la longueur des chaînes d'acide lactique produites. Cette concentration est maximale lorsque la longueur moyenne des chaînes est de 2 unités. Elle se réduit au fur et à mesure que la longueur des chaînes augmente. La cinétique de formation du lactide et sa distillation sont donc liées à la longueur des chaînes de l'oligomère d'acide lactique.

D'autre part la synthèse d'acide polylactique par ouverture de cycle doit se faire au départ d'un lactide très pure dont un des paramètres essentiels est la faible teneur en acidité libre. Le document WO 2005/056509 divulgue que le poids moléculaire de l'acide polylactique est d'autant plus élevé que l'acidité résiduelle du lactide, au départ duquel se fait la polymérisation, est faible.

Pour produire un polymère de haut poids moléculaires (>50000 daltons), l'acidité libre doit être inférieure à 20 meq/kg, préférentiellement inférieur à 5 meq/kg.

Or il est connu que l'acidité résiduelle du lactide sera d'autant plus faible que la longueur des chaînes de l'oligomère d'acide lactique, dont est issu le lactide par dépolymérisation, est grande. Pour ce faire, il est possible d'utiliser des chaînes de longueurs moyennes supérieures à 5 unités d'acide lactique, de préférence de 10 à 30 unités d'acide lactique, en présence d'un catalyseur d'estérification pour augmenter la cinétique de formation du lactide. L'utilisation de catalyseurs tels que les oxydes, les halogeno- et les organo- métaux des groupes 12 (ex Zn), 13 (ex : Al) et 14 (ex : Sn) du tableau des éléments sont bien connus et employés industriellement.

Des anti-oxydants peuvent être ajoutés au milieu réactionnel pour éviter la dégradation thermique de l'acide lactique et la formation de sous-produits généralement colorés. Parmi ceux-ci, on peut citer l'Ultranox 626, le trialkylphosphite, des mélanges d'aryl/alkylphosphite et des composés phénoliques.

Dans le cadre de la cyclisation et de la distillation du lactide par une technologie de couche mince, c'est à dire dans un réacteur maximisant la surface de vaporisation par rapport au volume de liquide, les catalyseurs généralement décrits pour la production de lactide sont des poussières d'étain ou de zinc, des chlorures d'étain ou de zinc, des sels organiques d'étain ou de zinc et d'acides organiques contenant entre 1 et 20 atomes de carbone. Ces catalyseurs ne sont pas entièrement satisfaisant pour l'obtention de lactide à partir d'un oligomère d'acide lactique.

En effet, l'étain divalent (Sn²⁺) utilisé sous forme d'oxyde augmente peu la cinétique de cyclisation et s'oxyde rapidement en Sn⁴⁺ beaucoup moins réactif. Lorsque l'étain divalent est utilisé sous la forme d'organo-étain, comme l'octanoate d'étain, celui-ci se décompose rapidement à haute température (>200°C), s'oxyde en Sn⁴⁺ et perd sa réactivité. Cette décomposition est accompagnée d'une racémisation de l'acide lactique et de la formation de composés colorés. De plus, la partie organique telle que l'acide 2 éthyl hexanoique peut être entraînée dans la phase vapeur et contaminer le lactide.

Quant aux halogeno-étains, tel que par example le SnCl₂, leur pouvoir catalytique est également faible et ils s'oxydent rapidement en Sn⁴⁺. De plus l'anion halogénure tel que par exemple le Cl⁻ est très corrosif et demande des équipements particuliers tel que des réacteurs vitrifiés.

Il existe donc un besoin pour un catalyseur qui augmente la cinétique de formation du lactide à partir d'oligomères d'acide lactique exempt des désavantages mentionnés ci-dessus.

L'objet de la présente invention est de fournir un procédé d'obtention de lactide à partir d'oligomères d'acide lactique en présence d'un catalyseur ayant une bonne cinétique de réaction.

Un autre objet de l'invention est de fournir un procédé d'obtention de lactide en présence d'un catalyseur stable à l'oxydation et à la dégradation à haute température (≥240°C).

Un autre objet de l'invention est de fournir un procédé d'obtention de lactide utilisant un catalyseur ne libérant pas de composés volatiles tels que par exemple l'acide 2 éthyl hexanoique.

Un autre objet de l'invention est de fournir un procédé d'obtention de lactide avec un taux de racémisation inférieur ou égal à 4%, de préférence inférieur ou égal à 1%.

Encore un autre objet de l'invention est de fournir un procédé qui permet d'obtenir un lactide ayant la plus faible coloration possible.

Enfin, un autre objet de l'invention est de fournir un procédé qui permet de limiter la présence d'impuretés provenant de réactions de dégradation.

La présente invention fournit un procédé d'obtention de lactide au départ d'oligomères d'acide lactique, le procédé comprenant les étapes suivantes:
(a) chauffer un oligomère d'acide lactique en présence d'un catalyseur à une température comprise entre 150°C et 300°C sous une pression inférieur à 0,01 MPa,
(b) distiller le lactide issu de l'étape (a),
(c) condenser et récupérer le lactide
caractérisé en ce que le catalyseur est un un sel métallique de l'anion phosphite (PO₃)³⁻ dans lequel le métal est choisi dans le groupe consistant en l'étain, l'aluminium, le zinc, le titane et le zirconium.

L'oligomère d'acide lactique peut être un oligomère de formule générale HO-[CHCH3-COO]n-H dans lequel n est compris entre 2 et 30. De préférence, n est compris entre 10 et 30.

L'oligomère d'acide lactique est mélangé avec le catalyseur par tout moyen approprié par exemple à l'aide d'un échangeur/mélangeur statique.

De préférence, le catalyseur est un phosphite métallique dans lequel le métal est choisi dans le groupe consistant en l'étain et le zinc. On entend par phosphite métallique, le sel métallique de l'anion (PO₃)³⁻. Plus préférablement, le catalyseur est un phosphite métallique dans lequel le métal est l'étain divalent. Encore plus préférablement, le phosphite d'étain divalent est le SnHPO₃

Le catalyseur peut être utilisé à la concentration comprise entre 0.1 et 10% poids, de préférence entre 0.1 et 5% poids, plus préférentiellement entre 0.1 et 3% poids.

La réaction peut se faire dans n'importe quel type de réacteur adapté à la production de lactide. Un réacteur d'évaporation est préféré car dès que le lactide est formé, il peut être distillé. Des exemples de réacteurs de ce type sont décrits dans le Perry's Chemical Engineer's Handbook, 7th édition, chapitre 11, pages107 à 118. Parmi ceux-ci, on peut citer l'évaporateur forcé, le short-path, l'évaporateur tubulaire, l'évaporateur à film tombant, l'évaporateur à couche mince, l'évaporateur flash ou l'évaporateur à disque.

La réaction est réalisée à une température comprise entre 150°C et 300°C, de préférence entre 230°C et 280°C et sous une pression inférieure à 0,01 MPa (100 mbar), de préférence inférieure à 0,005 MPa (50 mbar), plus préférablement inférieure à 0,002 MPa (20 mbar).

Le lactide obtenu dans le réacteur est sous forme vapeur. Il peut être ensuite optionellement distillé à une température supérieure à 150°C et une pression inférieure à 0,005 MPa (50 mbar) puis condensé pour produire un brut de lactide comprenant du lactide, du méso-lactide et des impuretés telles que l'acide lactique, les dimères, trimères, tétramères et pentamères d'acide lactique.

Le brut de lactide peut être ultérieurement purifié par toute technique connue de l'homme de l'art telle que par cristallisation en milieu fondu, par distillation ou par recristallisation par solvant afin d'obtenir un lactide d'une pureté suffisante pour, par exemple, la synthèse du polylactide par ouverture de cycle.

### Exemples

### 1. Oligomérisation de l'acide lactique

L'oligomérisation d'une solution d'acide lactique à 90 % en poids a été réalisée dans un réacteur agité en verre d'une contenance de 5 litres. L'eau a été distillée à 145°C et 0,02 MPa (200 mbar). Un reflux à 70°C a été installé entre le réacteur et le condenseur de manière à récupérer une partie de l'acide lactique entraîné dans la phase vapeur.

Après 8h de réaction, le vide a été fixé à 0,008 MPa (80 mbar) et la réaction d'estérification est poursuivie jusqu'à atteindre une acidité libre de 10% et une acidité totale de 122%. La pureté stéréospécifique de l'oligomère d'acide lactique obtenu est de 98,5%.

### 2. Cyclisation du lactide en ballon

L'oligomère obtenu ci-dessus a été introduit dans un ballon, puis chauffé à 250°C et maintenu sous agitation. Le phosphite d'étain, le SnHPO₃ utilisé comme catalyseur et commercialisé sous le nom TIB KAT 50 par la société Goldschmidt, a été ensuite introduit dans le ballon. Divers essais ont été réalisés en faisant varier la concentration en catalyseur (1%, 0,49%, 0,24% et 0,12%). A titre d'exemple comparatif, l'octanoate d'étain fréquemment utilisé comme catalyseur pour l'obtention de lactide au départ de l'oligomère de l'acide lactique a été utilisé.

Le ballon a été surmonté d'un reflux à 200°C et ensuite d'un condenseur refroidit à 70°C et enfin d'un ballon de récolte des condensats. Le tout a été mis sous vide entre 0,001 et 0,002 MPa (10 et 20 mbar).

La réaction a été maintenue durant 10 minutes à 250°C. Le brut de lactide condensé et le résidu d'oligomère ont été pesés et analysés soit par chromatographie en phase gazeuse après sylilation des composés carboxylés afin de déterminer les teneurs des divers constituants, soit par titration à l'aide d'hydroxyde de tetrabutylammonium 0,1 M du lactide dissous dans une solution d'acetonitrile afin de déterminer l'acidité libre et acidité totale. Les résultats des essais sont présentés dans les tableaux I et II.

La cyclisation réalisée avec le phosphite d'étain permet l'obtention d'un brut de lactide avec une teneur moindre en méso-lactide. Comparé à l'octanoate d'étain, l'utilisation du phosphite d'étain comme catalyseur entraîne donc une racémisation moindre de l'oligomère de l'acide lactique. Etant donné que l'oligomère de départ a une stéréospécificité de 98,5%, le minimum théorique de meso-lactide observable en absence de racémisation dans le brut de lactide est de 3%. La racémisation est calculée par la formule suivante : [(% de meso-lactide/2)- (100- pureté stéréospécifique de l'oligomère exprimée en %)]. Le tableau II divulgue que les synthèses réalisées en utilisant le phosphite d'étain mènent à des taux de racémisation inférieurs à 1% contrairement à celui observé (4,1%) lorsque la synthèse se fait en présence d'octanoate d'étain.

Le brut de lactide ne contient pas d'acide 2-éthylhexanoïque lorsque les cyclisations sont réalisées avec le phosphite d'étain.

La coloration du brut de lactide a été mesurée selon la norme ISO 6271-1:2004(F) et exprimée en unités Hazen.

Le brut de lactide produit avec le phosphite d'étain est blanc (coloration de 20 Hazen) comparé à un brut de lactide de couleur jaunâtre (coloration de 120 Hazen) lorsque l'octanoate d'étain est utilisé comme catalyseur.

**Tableau I**

| | Oligomère Initial | Rendement (L-Lactide/oligomère initial) | Oligomère résiduel | | | Brut de lactide formé | |
|---|---|---|---|---|---|---|---|
| | poids gr. | %poids | poids gr. | AT/AL % | L-acide lactique % poids | poids gr. | L-lactide %poids |
| (Comparatif) Sn Octanoate 1 %poids (Sn²⁺: 0,29%) | 200 | 43 | 98.2 | 121,5/4,5 | 84.5 | 101,8 | 85,2 |
| (Exemple 1 selon invention) SnHPO₃. 0.49% poids (Sn²⁺: 0,29%) | 207 | 43.3 | 106,6 | 123,1/4.5 | 92,7 | 100,4 | 89,3 |
| (Exemple 2 selon invention) SnHPO₃ 0,24% poids (Sn²⁺: 0,14%) | 197 | 37.3 | 115,2 | 123,1/5,1 | 97 | 81,8 | 89,9 |
| (Exemple 3 selon invention) SnHPO₃ 0,12% poids (Sn²⁺: 0,07%) | 204 | 17.9 | 161 | 123,1/5,7 | 97,4 | 43 | 84.9 |

| | | | | | | | |
|---|---|---|---|---|---|---|---|
| AL: acidité libre % AT: acidité totale % | | | | | | | |

**Tableau II**

| Brut de lactide Composition (% poids) | Octanoate d'étain (comparatif) | SnHPO₃ (invention) | SnHPO₃ (invention) | SnHPO₃ (invention) |
|---|---|---|---|---|
| | 1 % | 0.49% | 0.24% | 0,12% |
| L-Lactide | 85.2 | 89.3 | 89.9 | 84.9 |
| Meso-Lactide | 11,2 | 4.4 | 4.5 | 3.3 |
| Acide lactique | 1,8 | 2.5 | 2.5 | 6 |
| Dimère d'acide lactique | 0,8 | 1,9 | 1.6 | 3 |
| Trimère d'acide lactique | 0.2 | 1 | 0,8 | 1,9 |
| Tétramère d'acide lactique | 0,1 | 0,5 | 0,3 | 0,5 |
| Pentamère d'acide lactique | 0 | 0,1 | 0,1 | 0,1 |
| Acide 2-ethylhexanoique | 0.6 | 0 | 0 | 0 |
| Lactide+eau | 0,1 | 0,3 | 0.3 | 0.3 |
| Racémisation % | 4,1 | 0,7 | 0,75 | 0,15 |

### 3. Stabilité du catalyseur à la chaleur

Une première cyclisation a été réalisée comme décrit ci-dessus. Ensuite, lors du premier recyclage du phosphite d'étain, le résidu de la première cyclisation a été complété avec de l'oligomère frais (nouvellement introduit) et une deuxième cyclisation a été réalisée.

Lors du deuxième recyclage du phosphite d'étain, le résidu de la deuxième cyclisation a été complété avec de l'oligomère frais et une troisième cyclisation a été réalisée.

Les résultats sont repris dans le tableau III. II n'y a aucune perte de rendement de la cyclisation après plusieurs cyclisations successives en gardant le même catalyseur initial (ajout d'oligomère frais au résidu de la cyclisation précédente). Le phosphite d'étain conserve donc son activité catalytique.

**Tableau III**

| | Rendement (L-Lactide/oligomère initial) | Oligomères résiduels | Brut de lactide | L-Lactide |
|---|---|---|---|---|
| | % | gr. | gr. | %poids |
| SnHPO₃ 0,49% poids | 43,68 | 105 | 101 | 86,5 |
| Premier recyclage du SnHPO₃ | 42,7 | 106 | 98 | 87,1 |
| Deuxième recyclage du SnHPO₃ | 43,8 | 107 | 98 | 89,4 |

### 4. Cyclisation du lactide en couche mince

L'oligomère obtenu précedement (cfr point 1) est mélangé avec le catalyseur et alimenté à 0,8 kg/h dans un réacteur couche mince agité en verre de 50 cm². Le temps de séjour est de 10 minutes. La double enveloppe du réacteur est chauffée à 260°C et le réacteur est mis sous vide à 0,001 MPa (10 mbar). Les vapeurs de brut produites sont récupérées par condensation à 80 °C.

Le tableau IV indique que l'utilisation du phosphite d'étain comme catalyseur permet d'obtenir un rendement supérieur par rapport à celui obtenu avec l'octanoate d'étain et ce pour une même concentration d'étain: +/- 0,88%. La qualité du brut de lactide obtenue par catalyse avec le phosphite d'étain est de plus bien supérieure à celle obtenue par catalyse avec l'octanoate d'étain. Ceci est du à l'absence de distillation d'acide 2-ethyl hexanoique et à l'absence de racémisation.

**Tableau IV**

| Brut de lactide Composition (% poids) | Octanoate d'étain (comparatif) | SnHPO₃ (invention) |
|---|---|---|
| | 3% | 1,5% |
| L-Lactide | 89.2 | 93,9 |
| Meso-Lactide | 5 | 3 |
| Acide lactique | 1,8 | 1,9 |
| Dimère d'acide lactique | 0,6 | 0,7 |
| Trimère d'acide lactique | 0,2 | 0,2 |
| Tétramère d'acide lactique | 0,1 | 0.1 |
| Pentamère d'acide lactique | 0 | 0 |
| Acide 2-ethylhexanoique | 3 | 0 |
| Lactide+eau | 0,1 | 0,2 |
| Coloration (hazen) | 120 | 14 |
| Rendement ((L-Lactide/oligomère initial)) | 82% | 87% |
| Racémisation % | 1 | 0 |

## Revendications

1. Procédé d'obtention de lactide au départ d'oligomères d'acide lactique, le procédé comprenant les étapes suivantes:
(a) chauffer un oligomère d'acide lactique en présence d'un catalyseur à une température comprise entre 150°C et 300°C sous une pression inférieure à 0,01 MPa,
(b) distiller le lactide issu de l'étape (a),
(c) condenser et récupérer le lactide
**caractérisé en ce que** le catalyseur est un sel métallique de l'anion phosphite (PO₃)³⁻ dans lequel le métal est choisi dans le groupe consistant en l'étain, l'aluminium, le zinc, le titane et le zirconium.

2. Procédé selon la revendication 1 **caractérisé en ce que** le catalyseur est un sel métallique de l'anion phosphite (PO₃)³⁻ dans lequel le métal est l'étain divalent.

3. Procédé selon la revendication 2 **caractérisé en ce que** le catalyseur est le SnHPO₃.

4. Procédé selon les revendications 1 à 3 **caractérisé en ce que** le catalyseur est présent à la concentration comprise entre 0.1 et 10% poids.

5. Procédé selon la revendication 4 **caractérisé en ce que** la concentration est comprise entre 0.1 et 5% poids.

6. Procédé selon la revendication 5 **caractérisé en ce que** la concentration est comprise entre 0.1 et 3% poids.

7. Procédé selon la revendication 1 **caractérisé en ce que** l'oligomère d'acide lactique est de formule HO-[CHCH3-COO]ₙH. dans laquelle n est compris entre 2 et 30.

8. Procédé selon la revendication 7 **caractérisé en ce que** n est compris entre 10 et 30.

9. Procédé selon l'une quelconque des revendications précédentes **caractérisé en ce que** l'étape a) se fait à une pression inférieure à 0,002 MPa.

## Claims

1. A method for obtaining lactide starting with oligomers of lactic acid, the method comprising the following steps:
(a) heating a lactic acid oligomer in the presence of a catalyst at a temperature comprised between 150°C and 300°C under a pressure below 0.1 MPa,
(b) distilling the lactide from step (a),
(c) condensing and recovering the lactide
**characterized in that** the catalyst is a metal salt of the phosphite anion (PO₃)³⁻ wherein the metal is selected from the group consisting in tin, aluminium, zinc, titanium and zirconium.

2. The method according to claim 1, **characterized in that that** the catalyst is a metal salt of the phosphite anion (PO₃)³⁻ wherein the metal is divalent tin.

3. The method according to claim 2, **characterized in that** the catalyst is SnHPO₃.

4. The method according to claims 1 to 3 **characterized in that** the catalyst is present at the concentration comprised between 0.1 and 10% by weight.

5. The method according to claim 4, **characterized in that** the concentration is comprised between 0.1 and 5% by weight.

6. The method according to claim 5, **characterized in that** the concentration is comprised between 0.1 and 3% by weight.

7. The method according to claim 1, **characterized in that** the lactic acid oligomer is of formula HO-[CHCH₃-COO]ₙH, wherein n is comprised between 2 and 30.

8. The method according to claim 7, **characterized in that** n is comprised between 10 and 30.

9. The method according to any of the preceding claims, **characterized in that** step (a) is accomplished at a pressure below 0.002 MPa.

## Patentansprüche

1. Verfahren zur Herstellung von Lactid aus Oligomeren der Milchsäure, wobei das Verfahren die folgenden Schritte umfasst:
(a) Erhitzen eines Oligomers der Milchsäure bei Anwesenheit eines Katalysators auf eine Temperatur zwischen 150 °C und 300 °C inklusive bei einem Druck unter 0,01 MPa,
(b) Destillation des in Schritt (a) hergestellten Lactids,
(c) Kondensation und Gewinnung des Lactids,
**dadurch gekennzeichnet, dass** der Katalysator ein Metallsalz des Phosphitanions (PO₃)³⁻ ist, wobei das Metall aus der Gruppe ausgewählt ist, die aus dem Zinn, dem Aluminium, dem Zink, dem Titan und dem Zirkonium besteht.

2. Verfahren nach Anspruch 1, **dadurch gekennzeichnet, dass** der Katalysator ein Metallsalz des Phosphitanions (PO₃)³⁻ ist, wobei das Metall das zweiwertige Zinn ist.

3. Verfahren nach Anspruch 2, **dadurch gekennzeichnet, dass** der Katalysator das SnHPO₃ ist.

4. Verfahren nach den Ansprüchen 1 bis 3, **dadurch gekennzeichnet, dass** der Katalysator in der Konzentration zwischen 0,1 und 10 Gew.-% inklusive vorhanden ist.

5. Verfahren nach Anspruch 4, **dadurch gekennzeichnet, dass** die Konzentration zwischen 0,1 und 5 Gew.-% inklusive ist.

6. Verfahren nach Anspruch 5, **dadurch gekennzeichnet, dass** die Konzentration zwischen 0,1 und 3 Gew.-% inklusive ist.

7. Verfahren nach Anspruch 1, **dadurch gekennzeichnet, dass** das Oligomer der Milchsäure die Formel HO-[CHCH3-COO]ₙH. hat, wobei n zwischen 2 und 30 inklusive ist.

8. Verfahren nach Anspruch 7, **dadurch gekennzeichnet, dass** n zwischen 10 und 30 inklusive ist.

9. Verfahren nach einem der vorhergehenden Ansprüche, **dadurch gekennzeichnet, dass** der Schritt a) bei einem Druck unter 0,002 MPa erfolgt.
